Europäisches Patentamt

European Patent Office

Office européen des brevets

(19)

(11) Publication number: **0 070 127**
**A2**

(12) **EUROPEAN PATENT APPLICATION**

(21) Application number: **82303476.4**

(22) Date of filing: **02.07.82**

(51) Int. Cl.³: **A 61 K 9/20**
**A 61 K 47/00**

(30) Priority: **10.07.81 GB 8121302**

(43) Date of publication of application:
**19.01.83 Bulletin 83/3**

(84) Designated Contracting States:
**CH DE FR GB IT LI NL**

(71) Applicant: BEECHAM GROUP PLC
Beecham House Great West Road
Brentford Middlesex(GB)

(72) Inventor: Wells, James Ingram
Ridgeways New Town High Street
Wingham Kent(GB)

(72) Inventor: Khan, Karrar Ahmed
1 Ravensfield Gardens
Ewell Surrey(GB)

(74) Representative: Russell, Brian John et al,
European Patent Attorney Beecham Pharmaceuticals
Great Burgh Yew Tree Bottom Road
Epsom Surrey, KT18 5XQ(GB)

(54) **Tablets.**

(57) A tablet contains a poorly compressible medicament and a plasticizer incorporated into the body of the tablet. The plasticizer reduces the need for high pressures during the manufacture of the tablets, and reduces the problems associated with capping and laminating of the tablet.

EP 0 070 127 A2

## TABLETS

This invention relates to tablets containing a
plasticizer.

The tablet is one of the most common, and useful,
medicament formulations available. Typically tablets
will contain the medicament, an excipient to act as a
bulking agent, a binder to hold the tablet together,
a disintegrant to promote break up of the tablet after
administration to release the medicament, and a lubricant
to prevent tablet ingredients from sticking to the
tablet-punch. Also, it is conventional to film coat
tablets for a number of reasons, such as to improve
elegance, to add colour, to alter water permeability
and the like.

It is known that tablets containing poorly compressible ingredients can have the disadvantage that unduly
high pressures need to be used during their manufacture
to obtain a standard hardness of tablet, and that these
pressures can lead to problems with capping (top of
tablet breaking) and laminating (layers of tablet
breaking off).

In one known way of trying to overcome this tableting
problem with poorly compressible ingredients, higher than
usual levels of binder are used. However the resultant
tablets often have unacceptable disintegration times.

It is an object of this invention to reduce these disadvantages of known tablets containing poorly compressible ingredients.

It has been found that this object can be achieved by the incorporation into the body of the tablet of a minor proportion of a plasticizer.

Hitherto, plasticizers have been used in film coats for tablets, but it is believed that they have never been used in the manner of this invention.

Accordingly the present invention provides a tablet which comprises a poorly compressible ingredient and a plasticizer incorporated into the body of the tablet.

The skilled man is fully familiar with the term 'poorly compressible', in this context. He will either, from his general knowledge, be able to identify a given ingredient as 'poorly compressible', or he will merely carry out a routine compression test on a standard tablet formulation including the ingredient. Poorly compressible ingredients will give a tablet mix that caps or laminates under standard tableting pressure in this test.

The proportion of ingredient in the tablet is not critical, except that, of course, sufficient ingredient must be present so that its poor compressibility property, in the absence of the plasticizer, would cause it to affect the physical properties of the tablet. Thus as a general guide, the ingredient will represent at least 50% by weight of the tablet, more suitably at least 70% of the tablet.

Advantageously, the poorly compressible ingredient will be a medicament. In such cases, the skilled man will also appreciate that as there are finite limits on the size of a tablet, it will normally be the case that the medicament used will be a high dose medicament. High dose medicaments usually are present in tablets in a weight of at least 250 mg., for example 250 mg to 1000 mg.

Suitable examples of medicaments for use in the invention as poorly compressible ingredients will be readily apparent to the skilled man, and include paracetamol;non-steroidal anti-inflammatories such as phenyl butazone, naproxen, phenacetin and 4-(6'-methoxy-2'-naphthyl)-butan-2-one; vitamin preparations; ibuprofen; and the like.

The term 'plasticizer' is well known in the art, and materials that are plasticizers will, therefore, be readily recognised by the skilled man.

Suitable examples of plasticizers for use in this invention include propylene glycol, liquid polyethylene glycols such as the 200, 300, 400 and 600, glycerine, hexylene glycol, 1,2,6-trihydroxyhexane and solketal. When a non-aqueous system is used in the preparation of the tablet (see below), then any of the above plasticizers are suitable, as well as diethyl phthalate.

The weight of plasticizer in the tablet will be governed by the self-evident requirements that too little plasticizer will not give a meaningful effect, while too much plasticizer will in itself interfere with the tablet's compression properties (by way of example, very often the plasticizer will be a liquid, and so use of too much plasticizer would give excess liquid in the tablet).

- 4 -

Thus we have found that most suitably 0.1 to 2% (by weight of the tablet) plasticizer is incorporated, most suitably about 0.5%.

A preferred plasticizer is propylene glycol, due to its high level of activity coupled with low level of toxicity.

Suitable examples of binders for use in the tablets of this invention will be readily apparent to the skilled man. Examples include PVP, gelatin, methylhydroxyethyl cellulose, hydroxypropylmethyl cellulose, hydrolysed gelatin. Often the binder will be water soluble to avoid interfering with drug release after administration. Alternatively, the binder can be a water insoluble binder, such as ethyl cellulose, which would yield a sustained release tablet.

The level of incorporation of binder into the tablet will be as conventional, for example 0.1 to 10% by weight of the tablet, more suitably 2 to 5% of the tablet.

It will be appreciated that the tablets of the invention may contain the usual additional ingredients such as excipients, disintegrants and lubricants, and may be film coated.

From the aforesaid, it will be appreciated that preferred tablets of this invention comprise a poorly compressible high dose ingredient as at least 50% of the tablet, a water soluble binder as 2 to 5% of the tablet and a plasticizer as 0.1 to 2% of the tablet. (The % figures are based on wt/wt of the tablet).

Suitable and preferred examples of the variables in such tablets are as described hereinbefore.

This invention also provides a process for the preparation of the tablets of this invention, which process comprises mixing the plasticizer with the poorly compressible ingredient prior to compression, and then compressing the materials into tablet form.

It will be immediately appreciated by the skilled man that to ensure the relatively uniform distribution of ingredient and the plasticizer in the body of the tablet necessary to give a corresponding uniformity of physical properties of the tablet, it will normally be convenient to add the plasticizer in solution to the ingredient. A preferred solvent for making this solution is water, although organic solvents such as those used conventionally in granulation, such as acetone and isopropylalcohol may be used.

It is, therefore, often convenient to add the plasticizer to a solution of the binder (binders are usually incorporated in this way), and then to add the resultant solution to the ingredient (which will often be a medicament as hereinbefore discussed).

After this addition, the wet mix can be formed into a suitable dry powder/granulate in any of the conventional methods, such as wet granulation, spray granulation and spray drying. [ It will be appreciated that direct compression and pre-compression (slugging) techniques are not applicable to this invention, as they would not yield the necessary dispersion of the plasticizer in the ingredient.]

Any other tablet ingredients may be incorporated in conventional manner, for example either by mixing or by incorporating in the binder/plasticizer solution, at any suitable time prior to compression into the final tablet form.

The tablets of the invention are then prepared by compression.

The tablets, so formed, may then be film coated if so desired.

The tablets will be administered so as to give the therapeutically useful amount of the medicament.

The tablets of this invention have the advantage that they have improved compression properties, enabling for example harder tablets to be prepared at lower pressures than would be possible without the plasticizer, and they have a reduced tendency to cap and laminate.

The following Examples illustrate the invention.

## Example 1

| Ingredients | Weight | % Tablet |
|---|---|---|
| Paracetamol | 476.5 g | 95.3 |
| Explotab (sodium starch glycollate) | 10.0 | 2 |
| Kollidon 90 (polyvinyl pyrrolidone) | 10.0 | 2 |
| Propylene glycol | 1.0 | 0.2 |
| Magnesium stearate | 2.5 | 0.5 |

## Granulation

The paracetamol and the explotab (disintegrant) were blended in a tumbler mixer for 5 minutes. The Kollidon 90 (binder) was then added as a 8% w/v aqueous solution, containing the propylene glycol (plasticizer), and the resultant mixture wet massed in a planetary mixer for 10 minutes. Then the resultant mixture was wet screened through a 10 # (1.7 mm) screen, dried overnight at $50^{\circ}C$, and then dry screened through a 16 # (1.0 mm) screen.

## Tableting

A 1000-200 µm sieve cut of the granules was blended with the magnesium stearate (lubricant) for 5 minutes, and the resultant mixture tableted on a single punch tableting machine (Manesty F3), available from Manesty Machines Ltd, Speke, England, using 8 mm flat faced tooling at a compression weight of 200 mg.

The compression pressures used were as shown in Table 1 following, which pressures are in the conventional range.

## Comparison Tablets

The process detailed above was repeated but without the plasticizer, the medicament weight being increased to 477.5 g.

Example 2

Tableting Properties

The following parameters were examined:

(i)     Diametral crushing strength (Schleuniger Tester)
        [P Kp],

(ii)    Tensile strength ($\sigma$)

$$\sigma = \frac{2P}{\pi DT} \qquad MNm^{-2}$$

where D and T are the tablet diameters and
thickness.

(iii)   Friability
        % weight loss after 1000 revolutions,

(iv)    Disintegration time (BP 1973),

PARACETAMOL - Tablets with and without Plasticizers

| COMPRESSION PRESSURE $MNm^{-2}$ | 30 | | 60 | | 90 | | 120 | | 150 | |
|---|---|---|---|---|---|---|---|---|---|---|
| With plasticizer / Without | ✓ | ✓ | ✓ | ✓ | ✓ | ✓ | ✓ | ✓ | ✓ | ✓ |
| Crushing strength (Kp) | 0.59 | 2.29 | 3.87 | 2.74 | 8.38 | 3.61 | 11.19 | 5.95 | 11.93 | 6.32 |
| Tensile strength ($MNm^{-2}$) | 0.087 | 0.422 | 0.729 | 0.527 | 1.853 | 0.710 | 2.582 | 1.206 | 2.755 | 1.355 |
| Friability (%) | - | 12.90 | 6.09 | 10.23 | 3.91 | 8.78 | 3.96 | 10.27 | 3.62 | 10.35 |
| Disintegration time (min) | 0.217 | 0.100 | 0.217 | 0.100 | 0.483 | 0.130 | 0.800 | 0.170 | 1.700 | 0.300 |

These results show that the inclusion of the plasticizer greatly increases the strength of the tablets formed at conventional compression pressures, greatly decreases their friability, but at the same time does not have a detrimental effect on their disintegration.

CLAIMS

1.   A tablet which comprises a poorly compressible
     ingredient and a plasticizer incorporated into the
     body of the tablet.

2.   A tablet according to claim 1, in which the
     compressible ingredient represents at least 50% by
     weight of the tablet.

3.   A tablet according to claim 1 or claim 2, in which
     the compressible ingredient is a high dose medicament.

4.   A table according to any one of claims 1 to 3, in
     which the plasticizer is selected from proplyene
     glycol, liquid polyethylene glycol, glycerine,
     hexylene glycol, 1,2,6-trihydroxyhexane, solketal,
     and diethyl phthalate.

5.   A tablet according to any one of claims 1 to 4, in
     which from 0.1 to 2% plasticizer by weight of the
     tablet is incorporated.

6.   A tablet according to any one of claims 1 to 5, in
     which from 0.1 to 10% binder by weight of the tablet
     is incorporated.

7.   A tablet according to claim 1 comprising at least 50%
     by weight of a poorly compressible high dose medicament,
     from 2 to 5% by weight of a water soluble binder, and
     from 0.1 to 2% by weight of a plasticizer.

8.   A process for the preparation of a tablet according
     to claim 1, which comprises mixing the plasticizer
     with the poorly compressible ingredient prior to
     compression, and then compressing the materials into
     tablet form.